# EUROPÄISCHE PATENTSCHRIFT

(11) **EP 1 466 578 B1**
(45) Veröffentlichungstag und Bekanntmachung des Hinweises auf die Patenterteilung: **16.09.2009**
(21) Anmeldenummer: 03014895.1
(22) Anmeldetag: 01.07.2003
(51) Int. Cl.: A61Q 1/00, A61K 8/02, A61K 8/97

(54) **Trockenes Wattepad, enthaltend kosmetische Wirksubstanzen**
Dry cotton pad containing active cosmetic agent
Pad en coton sec comprenant des agents actifs cosmétiques

(30) Priorität: 09.04.2003 DE 20305717 U
(43) Veröffentlichungstag der Anmeldung: 13.10.2004
(73) Patentinhaber: W. Pelz GmbH & Co., 23812 Wahlstedt/Holstein (DE)
(72) Erfinder: Sasse, Uwe, 23795 Bad Segeberg (DE)
(74) Vertreter: Hauck Patent- und Rechtsanwälte

(56) Entgegenhaltungen:
- WO-A-01/03749
- WO-A-97/38677
- WO-A-20/04006869
- DE-A- 10 240 074
- DE-U- 20 103 768
- US-A- 5 919 152
- [Online] Gefunden im Internet: <URL:http://web.archive.org/web/20010618094 435/http://www.aiibeauty.com/andreaqs.html> [gefunden am 2006-07-17]
- [Online] Gefunden im Internet: <URL:http://web.archive.org/web/20040405181 600/http://folica.com/Andrea_EyeQ_s_E_d929. html> [gefunden am 2006-11-08]

## Beschreibung

Die Erfindung bezieht sich auf eine Wattescheibe.

Wattescheiben oder "Wattepads" werden insbesondere für kosmetische, hygienische und medizinische Zwecke eingesetzt.

Aus der DE 202 04 988 U1 ist eine Wattescheibe bekannt, bei der die Fasern in zumindest einer Oberflächenschicht mit einer wasserlöslichen Substanz beschichtet sind, welche im nassen Zustand den Verbund der Fasern aufrecht erhält und deren Wirkstoffe durch Benetzung mit Wasser zur Übertragung auf die jeweilige Oberfläche aktivierbar sind. Die für die Haftung und Freisetzung bzw. Aktivierung der Wirkstoffe in der Beschichtung vorgesehenen Anteile an Tensiden können bei der Anwendung stören.

Die DE 199 11 041 A1 offenbart flexible und saugfähige, mit kosmetischen oder dermatologischen Wirkstoffen oder Zubereitungen ausgerüstete Zellstoffpads, die Wirkstoffe oder Zubereitungen enthalten, die bei 40 °C fest sind, in Form von Nanopartikeln mit einem mittleren Teilchendurchmesser von weniger als 300 Nanometern. Die Nanopartikel können z.B. aus Wachsen, gegebenenfalls im Gemisch mit lipophilen, kosmetischen oder dermatologischen Wirkstoffen, Sterinen, Chitosanen oder anorganischen Oxiden bestehen. Der Einschluß in Nanopartikel kann die Freisetzung der kosmetischen Wirkstoffe verzögern. Die Ausrüstung der Wattescheiben mit den kosmetischen Wirkstoffen ist sehr aufwendig.

Davon ausgehend liegt der Erfindung die Aufgabe zugrunde, eine keine störenden Zusatzstoffe enthaltende Wattescheibe mit kosmetischer Wirkung zur Verfügung zu stellen.

Die Aufgabe wird durch eine Wattescheibe mit den Merkmalen des Anspruches 1 gelöst. Vorteilhafte Ausgestaltungen der Wattescheibe sind in den Unteransprüchen angegeben.

Die erfindungsgemäße Wattescheibe besteht im wesentlichen oder vollständig aus Baumwollfasern mit einem trockenen Auftrag enthaltend eine oder mehrere kosmetische Wirksubstanzen aus der Gruppe der Pflanzenextrakte, Provitamine und Vitamine.

Die reinen Baumwollfasern, aus denen die Wattescheibe im wesentlichen oder vollständig besteht, haben im Gegensatz zur glatten Kunstfaser eine Depotwirkung durch die natürliche Faserstruktur. In der Struktur haftet der kosmetische Wirkstoff und wird durch die von der Benutzerin aufgetragene Flüssigkeit herausgelöst und aktiviert. Diese Effekte stellen sich besonders bei den Wirksubstanzen aus der Gruppe der Pflanzenextrakte, Provitamine und Vitamine ein. Störende Zusatzstoffe fehlen. Hautbelastende Konservierungsstoffe sind nicht erforderlich, weil der Auftrag der kosmetischen bzw. pflanzlichen Wirksubstanzen, die grundsätzlich mikrobiologisch sehr anfällig sind, trocken ist. Der kosmetische Wirkstoff wird durch eine Flüssigkeit nach Wahl der Benutzerin herausgelöst.

Gemäß einer Ausgestaltung enthält der Auftrag Aloe vera als Pflanzenextrakte und/oder Pantenol als Provitamin.

Gemäß einer Ausgestaltung enthält der Auftrag Radikalfänger und/oder Liposomen und/oder Lecithine und/oder UV-Schutzfiltersubstanzen und/oder Anthocyane und/oder Duftstoffe und/oder andere pflegerische Bestandteile.

Gemäß einer Ausgestaltung ist der Auftrag eine getrocknete Lösung der mindestens einen kosmetischen Wirksubstanz in einer Flüssigkeit. Die kosmetische Wirksubstanz kann in verschiedenen Lösungsmitteln wie Wasser oder alkoholischen Lösungen sowie Dispersionen gelöst sein.

Gemäß einer Ausgestaltung ist der Auftrag eine getrocknete Lösung der mindestens einen kosmetischen Wirksubstanz in einer Flüssigkeit enthaltend einen Solubilisator. Der Solubilisator verankert die kosmetische Wirksubstanz und schafft ein angenehmes Anwendungsgefühl durch Verminderung des Reibwertes in der Wattescheibe auf der Haut.

Gemäß einer Ausgestaltung ist der Auftrag auf die Fasern in einer oder beiden Oberflächen und/oder auf die Fasern zwischen den Oberflächen aufgebracht. Die Oberflächen der Wattepads können so gezielt mit denselben oder verschiedenen spezifischen Wirkstoffen ausgerüstet sein.

Gemäß einer Ausgestaltung ist der Auftrag (ein- oder beidseitig) aufgesprüht und/oder durch Tränken aufgebracht und/oder mit Walzen aufgebracht und getrocknet.

## Patentansprüche

1. Wattescheibe im wesentlichen oder vollständig aus Baumwollfasern mit einem von Lipiden freien trockenen Auftrag enthaltend Aloe Vera und/oder Panthenol und wahlweise eine oder mehrere weitere Wirksubstanzen aus der Gruppe der Pflanzenextrakte, Provitamine und Vitamine, bei der der Auftrag eine getrocknete Lösung der mindestens einen kosmetischen Wirksubstanz in einer Flüssigkeit ist und der Auftrag aufgesprüht und/oder durch Tränken aufgebracht und/oder mit Walzen aufgebracht und getrocknet ist.

2. Wattescheibe nach Anspruch 1, bei der der Auftrag Radikalfänger und/oder Liposomen und/oder Lecithine und/oder UV-Schutzfiltersubstanzen und/oder Anthocyane und/oder Duftstoffe und/oder andere pflegerische Bestandteile enthält.

3. Wattescheibe nach Anspruch 1 oder 2, bei der der Auftrag keine Konservierungsstoffe enthält.

4. Wattescheibe nach einem der Ansprüche 1 bis 3, bei der der Auftrag eine getrocknete Lösung der mindestens einen kosmetischen Wirksubstanz in einer Flüssigkeit enthaltend einen Solubilisator ist.

5. Wattescheibe nach einem der Ansprüche 1 bis 4, bei der der Auftrag auf die Fasern in einer oder beiden Oberflächen und/oder auf die Fasern zwischen den Oberflächen aufgebracht ist.

## Claims

1. A cotton pad, made essentially or completely from cotton fibres with a dry coating free of lipids, containing aloe vera and/or panthenol and optionally one or plural further active agents selected from the group consisting of plant extracts, provitamins and vitamins, wherein the coating is a dried solution of the at least one cosmetic active agent in a liquid, and the coating is sprayed up and/or applied by soaking and/or is applied by rollers and dried.

2. A cotton pad according to claim 1, wherein the coating contains radical scavengers and/or liposomes and/or lecithins and/or UV-protection filtering agents and/or anthocyans and/or fragrants and/or other body care constituents.

3. A cotton pad according to claim 1 or 2, wherein the coating contains no preservatives.

4. A cotton pad according to any one of claims 1 to 3, wherein the coating is a dried solution of the at least one cosmetic active agent in a liquid that contains a solubilising agent.

5. A cotton pad according to any one of claims 1 to 4, wherein the coating is applied to the fibres in one or both surfaces and/or to the fibres between the surfaces.

## Revendications

1. Pad en coton, essentiellement ou entièrement de fibres de coton avec un revêtement sec libre de lipides, contenant aloe vera et/ou panthénol et au choix en outre un ou plusieurs autres agents actifs sélectionnés du groupe des extraits de plantes, provitamines et vitamines, dans lequel le revêtement est une solution séchée de l'au moins un agent actif cosmétique dans un liquide, et le revêtement est arrosé et/ou appliqué par imprégnation et/ou appliqué par des rouleaux et séché.

2. Pad en coton selon la revendication 1, dans lequel le revêtement contient des piégeurs de radicaux libres et/ou des liposomes et/ou des lécithines et/ou des agents de protection contre les rayons UV et/ou des anthocyanes et/ou des parfums et/ou d'autres composants du soin corporel.

3. Pad en coton selon la revendication 1 or 2, dans lequel le revêtement ne contient pas des agents de conservation.

4. Pad en coton selon l'une quelconque des revendications 1 à 3, dans lequel le revêtement est une solution séchée de l'au moins un agent actif cosmétique dans un liquide qui contient un agent de solubilisation.

5. Pad en coton selon l'une quelconque des revendications 1 à 4, dans lequel le revêtement est appliqué sur les fibres dans une ou les deux surfaces et/ou aux fibres entre les surfaces.
